# EUROPÄISCHE PATENTANMELDUNG

(11) **EP 1 285 927 A2**
(43) Veröffentlichungstag der Anmeldung: **26.02.2003**
(21) Anmeldenummer: 02014272.5
(22) Anmeldetag: 26.06.2002
(51) Int. Cl.: C07J 41/00, A61K 31/565, A61P 5/46, A61P 15/08

(54) **Verwendung von Glucocorticoid-Rezeptorantagonisten zur Vorbeugung und Behandlung von Erkrankungen des männlichen Reproduktionssystems**

(30) Priorität: 16.08.2001 DE 10140113; 27.08.2001 US 315099 P
(71) Anmelder: Jenapharm GmbH & Co. KG, 07745 Jena (DE)
(72) Erfinder: Patchev, Vladimir, 07749 Jena (DE); Sobek, Lothar, 07743 Jena (DE); Schubert, Gerd, 07743 Jena (DE)
(74) Vertreter: Störle, Christian, Dr.

(57) **Zusammenfassung**

Beschrieben wird die Verwendung von Glucocorticoid-Rezeptorantagonisten zur Vorbeugung und/oder Behandlung von durch verminderte Androgen-Produktion verursachte Erkrankungen sowie hierfür geeignete Glucocorticoid-Rezeptorantagonisten.

## Beschreibung

Die Erfindung bezieht sich auf die Verwendung von Glucocorticoid-Rezeptorantagonisten zur Vorbeugung und/oder Behandlung von Erkrankungen des männlichen Reproduktionssystems sowie auf insbesondere dazu geeignete Glucocorticoid-Rezeptorantagonisten.

Es ist bekannt, daß physischer und/oder psychischer Stress, Alter sowie exogene Faktoren, wie Drogen und übermäßiger Alkoholgebrauch, beim Mann zu sexuellen Dysfunktionen und Hypogonadismus führen können. Diese Krankheiten werden nach der derzeit herrschenden Auffassung auf eine verminderte Androgen-Produktion, insbesondere eine verminderte Testosteron-Produktion, zurückgeführt.

Es wurden verschiedene Versuche untemommen, vorstehende Erkrankungen zu behandeln. Die dabei eingesetzten Arzneimittel waren aber entweder nicht ausreichend wirksam, zeigten gravierende Nebenwirkungen, die die Patienten mehr schädigten als heilten, oder waren aus anderen Gründen wenig geeignet.

Es besteht deshalb ein ganz erheblicher Bedarf an Verbindungen zur Vorbeugung und/oder Behandlung von vorstehenden Krankheiten.

Der vorliegenden Erfindung liegt somit die Aufgabe zugrunde, die Symptome von durch eine verminderte Androgen-Produktion verursachte Erkrankungen vorbeugen und behandeln zu können.

Ziel der vorliegenden Erfindung ist es ferner, Verbindungen bereitzustellen, die in günstiger Weise zur Behandlung und/oder Vorbeugung von Erkrankungen eingesetzt werden können, die durch eine verminderte Androgen-Produktion verursacht werden.

Erfindungsgemäß wird vorgenannte Aufgabe durch die Verwendung von Glucocorticoid-Rezeptorantagonisten gelöst. Unter einem "Glucocorticoid-Rezeptorantagonisten" werden im Sinne der vorliegenden Erfindung Pharmaka verstanden, die die Wirkung von Glucocorticoiden durch Bindung an Glucocorticoid-Rezeptoren hemmen.

Die vorliegende Erfindung beruht auf der überraschenden Erkenntnis, daß bei Verabreichung von Glucocorticoid-Rezeptorantagonisten die Androgen-Produktion, die vorher durch Glucocorticoid-Überschuß vermindert war, erhöht wird.

Werden beispielsweise Leydig-Zellen (Zellen aus den Hoden, die männliche Geschlechtshormone produzieren) mit humanem Choriongonadotropin (hCG) stimuliert, erfolgt ein Anstieg der Testosteron-Produktion durch diese Zellen. Werden nun die Zellen mit hCG und einem Glucocorticoid, z.B. Dexamethason (ein Ligand für den Glucocorticoid-Rezeptor (GR)), inkubiert, ist eine signifikante Verminderung der Testosteron-Produktion zu beobachten. Es wurde nun gefunden, daß die Verminderung der Testosteron-Produktion durch das Glucocorticoid verhindert wird, wenn in einem solchen Experiment dieses Glucocorticoid zusammen mit einem Glucocorticoid-Rezeptorantagonisten gegeben wird. Dieser Effekt wurde nicht nur bei Zellen beobachtet, sondern auch bei Versuchstieren.

Streß oder erhöhte Glucocorticoid-Blutspiegel verursachen bei Versuchstieren eine Hemmung der endokrinen sekretorischen Aktivität der männlichen Gonaden, die durch erniedrigte Serum-Testosteronspiegel dokumentiert wird. Gleichzeitig wird eine Hemmung (Verminderung) der männlichen Sexualaktivität beobachtet. Diese Symptome sind unter anderem für den Hypogonadismus charakteristisch und werden auch bei anderen Krankheitsbildern beobachtet, wie beispielsweise Streß, insbesondere chronischem Streß.

Glucocorticoid-Rezeptorantagonisten, die natürliche oder synthetische Verbindungen sein können, belegen den Glucocorticoid-Rezeptor und verdrängen dabei den natürlichen (endogenen) Liganden der Glucocorticoid-Rezeptoren, die Glucocorticoide, so daß durch eine selektive Antagonisierung des Glucocorticoid-Rezeptors die Übertragung chemischer Signale über diesen Rezeptor zumindest vermindert, aber sogar auch nahezu vollständig verhindert werden kann. Durch die Verdrängung der Glucocorticoide durch Glucocorticoid-Rezeptorantagonisten wird die übermäßige Besetzung der Glucorticoid-Rezeptoren durch Glucocorticoide antagonisiert.

Diese Verminderung oder Verhinderung der Besetzung der Glucocorticoid-Rezeptoren durch Glucocorticoide kann insbesondere dann wünschenswert sein, wenn im Körper der Glucocorticoid-Spiegel erhöht ist. Eine solche Erhöhung kann beispielsweise hervorgerufen werden durch (i) physischen oder psychischen Streß, (ii) pathologische Erhöhung der sekretorischen Aktivität der Nebennierenrinde (iii) Alkohol- und Drogenmißbrauch und -entzug, (iv) exogene Verabreichung von Medikamenten, z.B. Cortisole, für die Behandlung von chronischen Erkrankungen und (v) Altern.

Faßt man die vorstehend geschilderten Erkenntnisse und experimentellen Ergebnisse zusammen, so lassen diese vermuten - ohne daran gebunden zu sein -, daß die übermäßige Sekretion von Glucocorticoiden in einer übermäßigen Besetzung der Glucorcorticoid-Rezeptoren in endokrinen Zellen der männlichen Gonaden und/oder in den relevanten Bereichen des Zentralnervensystems resultiert. Dies kann Störungen der Reproduktionsfunktion bei Männern hervorrufen, die verursacht sein können durch eine verminderte Produktion der männlichen Sexualhormone (Androgene, insbesondere Testosteron), eine Beeinträchtigung der Anspechbarkeit des endokrinen Systems der männlichen Gonaden für den stimulierenden Effekt von Gonadotropin, Beeinträchtigung der neuronalen Ansprechbarkeit für sexuelle Stimuli und demgemäß eine erektile Dysfunktion. Diese Symptome werden allgemein mit dem Ausdruck "Hypogonadismus beim Mann" beschrieben, die eine Vielzahl von somatischen und endokrinen Dysfunktionen umfaßt. Dabei wird unter dem Ausdruck "Hypogonadismus" im Sinne der vorliegenden Erfindung kein Hypogonadismus verstanden, der in Folge der operativen Entfernung (Gonadektomie) oder des angeborenen Fehlers (Agenesie) der männlichen Gonaden entstanden ist.

Durch Verabreichung von Glucocorticoid-Rezeptorantagonisten kann vor allem der Hypogonadismus beim Mann, insbesondere der hypogonadotroper Hypogonadismus, sexuelle Dysfunktionen beim Mann und Infertilität, besonders gut behandelt und/oder vorgebeugt werden, wobei vermutlich die übermäßige Besetzung der Glucocorticoid-Rezeptoren durch Glucocorticoid-Rezeptorantagonisten im Organismus, insbesondere in den für die Reproduktion verantwortlichen Organen und/oder den neuronalen Schaltkreisen, die für ihre Kontrolle verantwortlich sind, vermindert oder verhindert wird.

Die Wechselwirkung von Glucocorticoiden mit den Glucocorticoid-Rezeptoren kann nach zwei Mechanismen ablaufen. Der Typ-1-Mechanismus zeichnet sich durch eine Wechselwirkung des Glucocorticoid-Rezeptors mit speziellen DNA-Sequenzen aus, wohingegen beim Typ-2-Mechanismus die Wechselwirkung von Glucocorticoid-Rezeptoren mit anderen Transkriptionsfaktoren in Abwesenheit spezifischer DNA-Bindung, möglicherweise über eine direkte Protein/Protein-Intraktion, beteiligt ist.

In einer bevorzugten Ausführungsform antagonisiert der erfindungsgemäß verwendete Glucocorticoid-Rezeptorantagonist die Typ-1-Transkriptionsinduktion der Glucocorticoid-Rezeptor-Gene. Durch Verwendung von Glucocorticoid-Rezeptorantagonisten mit diesen Eigenschaften wird ein besonders guter therapeutischer Effekt bei der Behandlung der vorgenannten Erkrankungen beobachtet. Es ist bekannt, daß Glucocorticoide für das Immunsystem eine wichtige Rolle spielen. Da die Glucocorticoidvermittelte Immunsupression ausschließlich über den Typ-2-Mechanismus der Glucocorticoid-Rezeptor-Wirkung verläuft, sind Glucocorticoid-Rezeptorantagonisten bevorzugt, die die Typ-1-Transkriptionsinduktion der Glucocorticoid-Rezeptor-Gene antagonisieren, da dadurch keine negativen Auswirkungen auf das Immunsystem festgestellt werden konnten.

In einer anderen bevorzugten Ausführungsform antagonisiert der Glucocorticoid-Rezeptorantagonist die Typ-2-Transkriptionsinhibierung im wesentlichen nicht. Bei der erfindungsgemäßen Verwendung von Glucocorticoid-Rezeptorantagonisten mit dieser Eigenschaft konnten keine Nebenwirkungen, insbesondere nicht auf das Immunsystem, festgestellt werden. Dabei bedeutet der Begriff "im wesentlichen nicht", daß eine mögliche Antagonisierung nur in soweit festzustellen ist, daß keine nicht-physiologischen oder pathologischen Wirkungen beobachtet werden.

Vorzugsweise binden die für die erfindungsgemäße Verwendung eingesetzten Glucocorticoid-Rezeptorantagonisten im wesentlichen nicht an andere Steroid-Rezeptoren. Dabei bedeutet der Ausdruck "im wesentlichen nicht", daß nur soviel Glucocorticoid-Rezeptorantagonist an andere Steroid-Rezeptoren als Glucocorticoid-Rezeptoren bindet, daß keine durch die anderen Rezeptoren vermittelten Wirkungen festgestellt bzw. existierende physiologische Wirkungen aufgehoben werden können. Beispiele anderer Steroid-Rezeptoren sind die Mineralcorticoid-Rezeptoren, Östrogen-Rezeptoren, Progesteron-Rezeptoren, und Androgen-Rezeptoren. Durch diese hohe Selektivität der erfindungsgemäß verwendeten Glucocorticoid-Rezeptorantagonisten wird erreicht daß die erfindungsgemäß gewünschten Wirkungen besonders deutlich ausgeprägt sind und damit die erfindungsgemäß zu behandelnden Erkrankungen besonders gut behandelt werden können, auf der anderen Seite aber keine unerwünschten Nebenwirkungen, insbesondere solche die durch eine Belegung der anderen Steroid-Rezeptoren hervorgerufen werden, beobachtet werden.

Vorzugsweise erfolgt durch die erfindungsgemäß verwendeten Glucocorticoid-Rezeptorantagonist keine vollständige Hemmung von durch Glucocorticoid-Rezeptoren vermittelte Signale. In diesem Zusammenhang ist darauf hinzuweisen, daß zur Aufrechterhaltung grundlegender Vitalfunktionen und für eine adequate Anpassung an durch die Umgebung hervorgerufene Herausforderungen ein bestimmtes Niveau von durch Glucocorticoid-Rezeptoren vermittelter Signale notwendig ist. Aus diesen Gründen ist eine vollständige Hemmung von durch Glucocorticoid-Rezeptoren vermittelten Signale nicht erwünscht, insbesondere um unerwünschte Effekte der Unterbrechung des durch Glucocorticoide-vermittelten Feedbacks (d.h. iatrogene Glucocorticoid-Rezeptor-Resistenz) und unkontrollierte pituitäre-adrenale Hyperaktivität zu verhindern.

Zur erfindungsgemäßen Verwendung können die Glucocorticoid-Rezeptorantagonisten enteral oder parenteral, für Menschen insbesondere in einer Dosis von 0,001-100 mg pro kg Körpergewicht verabreicht werden. Der Glucocorticoid-Rezeptorantagonist kann zusammen mit pharmazeutisch geeigneten Hilfsmitteln verabreicht werden. Der Glucocorticoid-Rezeptorantagonist kann in eine feste Dosiseinheit verpresst sein, beispielsweise eine Tablette, oder in anderer Weise formuliert vorliegen, z.B. als Kapseln oder Suppositorien. Durch Verwendung von pharmazeutisch verträglichen Flüssigkeiten können die Glucocorticoid-Rezeptorantagonisten auch in Form von Lösungen, Suspensionen, Emulsionen, als Injektionspräparate, Tropfen, Sprays hergerichtet sein. Arzneimittel zur erfindungsgemäßen Verwendung können weiterhin Additive, wie Füllstoffe, Farbstoffe und polymere Binder enthalten. Dabei kann grundsätzlich jedes pharmazeutisch verträgliche Additiv verwendet werden, das mit der Funktion der erfindungsgemäß verwendeten Glucocorticoid-Rezeptorantagonisten nicht negativ interferiert. Geeignete Träger, mit der der Glucocorticoid-Rezeptorantagonist verabreicht werden kann, umfaßt Laktose, Stärke, Zellulosederivate und Gemische davon.

Bei Glucocorticoid-Rezeptorantagonisten kann es sich um Verbindungen handeln, die bekannt sind. Bei unbekannten Verbindungen kann in einfacher, dem Fachmann bekannter Weise getestet werden, ob sie die Eigenschaften eines Glucocorticoid-Antagonsiten besitzt. Dazu kann beispielsweise eine zu testende Verbindung zusammen mit einem Glucocorticoid in einem Testsystem für Glucocorticoide-Rezeptoren inkubiert werden und geprüft werden, ob in diesem Testsystem die durch Glucocorticoide vermittelte Wirkung in Anwesenheit des Antagonisten reduziert wird.

Glucocorticoid-Rezeptorantagonisten sind beispielsweise die in EP 0 683 172 A1 beschriebenen 11,21-Bisphenyl-19-norpregnan-Derivate der Formel 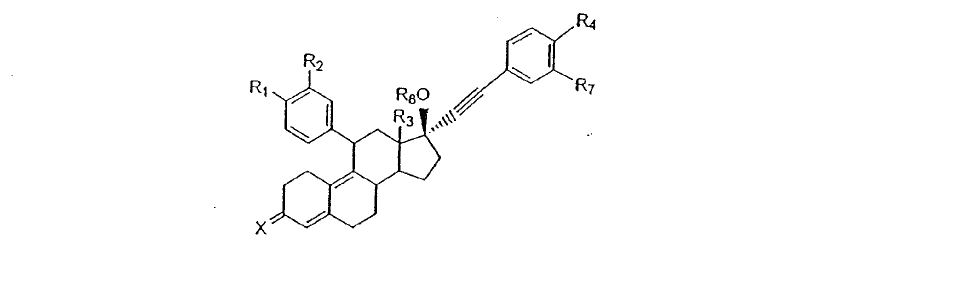 in der R₁ für Wasserstoff, Halogen, (1-6C)Alkoxy und NR₅R₆ steht, wobei R₅ und R₆ unabhängig voneinander ausgewählt sind aus Wasserstoff, (1-6C)Alkyl oder R₅ und R₆ zusammen ein (3-6C)Alkylen bilden, R₂ für Wasserstoff steht, oder R₁ und R₂ zusammen eine (1-3C)Alkylendioxygruppe bilden, die gegebenenfalls mit einem oder mehreren Halogenatomen substituiert ist, R₃ Methyl oder Ethyl bedeutet, R₄ aus C(O)-NR₅R₆, SOₙ-(1-6C)Alkyl, das gegebenenfalls mit einem oder mehreren Halogenatomen substituiert ist, SOₙ-(3-6C)Cycloalkyl, wobei n 1 oder 2 ist, SO₂-NR₅R₆, 2-Oxypyrrolidinyl und NR₅R₆ ausgewählt ist, R₇ für Wasserstoff oder (1-6C)Alkyl steht, R₈ Wasserstoff oder ein Carboxy-1-oxo(1-6C)-alkyl bedeutet und X aus (H, OH), O, und NOH ausgewählt ist. Diese sind für die erfindungsgemäße Verwendung besonders geeignet, weil die entsprechenden Erkrankungen besonders gut behandelt werden können.

Andere ebenfalls für die erfindungsgemäße Verwendung aufgrund der guten Behandelbarkeit vorgenannter Erkrankungen geeignete Glucocorticoid-Rezeptorantagonisten sind die in der EP 0 763 541 A1 beschriebenen 11-(Phenylsubstituierten)-estra-4,9-dien-Derivate der Formel 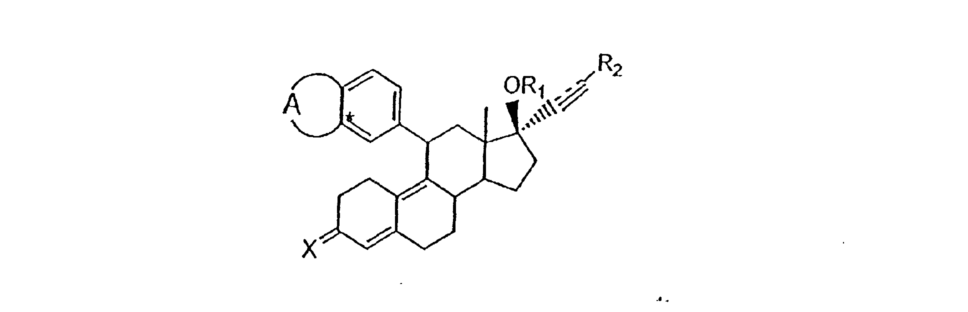 in der A den Rest eines 5- oder 6-gliedrigen Ringes mit 2 Heteroatomen darstellt, die nicht miteinander verbunden sind und unabhängig von O und S ausgewählt sind, wobei der Ring gegebenenfalls mit einem oder mehreren Halogenatomen substituiert ist, oder A einen 5- oder 6-gliedrigen Ring darstellt, der keine C-C-Doppelbindungen aufweist, der 1 Heteroatom ausgewählt aus O und S enthält, wobei das Heteroatom an die Phenylgruppe an der mit einem Stern markierten Position verbunden ist, wobei der Ring gegebenenfalls mit einem oder mehreren Wasserstoffatomen substituiert ist, R₁ für Wasserstoff oder 1-Oxo(1-4C)alkyl, R₂ für H, (1-8C)Alkyl, Halogen oder CF₃ stehen, X aus (H, OH,) O und NOH ausgewählt ist und die gestrichelte Linie eine gegebenenfalls vorliegende Bindung darstellt.

Andere Glucocorticoid-Rezeptorantagonisten, wie in WO 01/44267 beschrieben, können unter den in der folgenden Formel dargestellten Verbindungen gefunden werden. 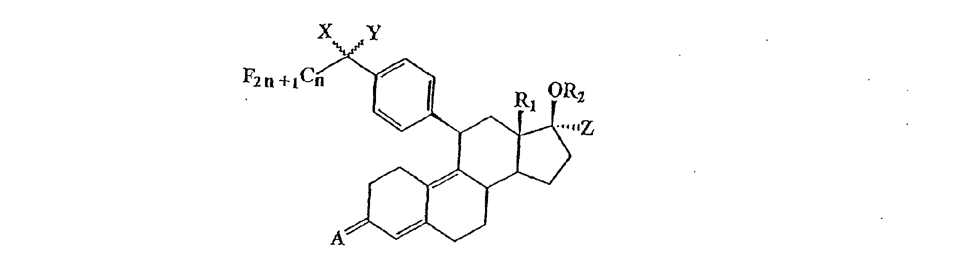 worin R₁ ein C₁₋₆-Alkylrest ist, R₂ ein Wasserstoffatom, ein C₁₋₆-Alkylrest oder ein C₁₋₆ Acylrest ist, X eine Hydroxylgruppe oder eine Trimethylsiloxy-Gruppe bedeutet und Y ein Wasserstoffatom oder eine Perfluoralkylgruppe der allgemeinen Formel CₙF₂ₙ₊₁ bedeutet oder X und Y zusammen eine Oxogruppe oder Oximino-Gruppe NOR₃ bedeuten, Z ein Wasserstoffatom, ein C₁₋₆-Alkylrest oder eine substituierte Methylengruppe CH₂W darstellt, wobei W eine Hydroxylgruppe, eine C₁₋₆-Alkoxygruppe, eine C₁₋₆-Alkylthiogruppe, ein Halogenatom oder ein Pseudohalogen darstellt, und A eine Oxogruppe, eine Oximino-Gruppe NOR₃, eine 1,3-Dithian- oder eine 1,3-Dithiolangruppe bedeutet, wobei R₃ ein Wasserstoffatom, einen C₁₋₈-Alkyl-, Aryl-, Alkylaryl- oder Arylalkylrest, einen C₁₋₈-Acylrest, einen Rest CONHR₄, COSR₄ oder COOR₄ bedeutet, wobei R₄ ein Wasserstoffatom oder einen C₁₋₈-Alkyl, Aryl-, Aaralkyl- oder Alkylarylrest bedeutet und n eine ganze Zahl von 1 bis 4 ist.

Als Glucocorticoid-Rezeptorantagonisten kommen ebenfalls in Frage die Verbindungen RU38486 (EP 0057 115) und KB 285 (WO 99/63976) gemäß den beiden nachfolgenden Strukturformeln

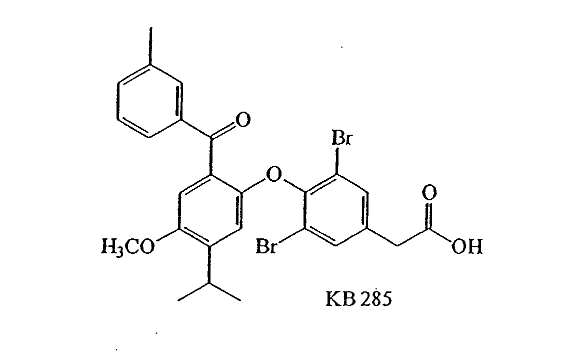

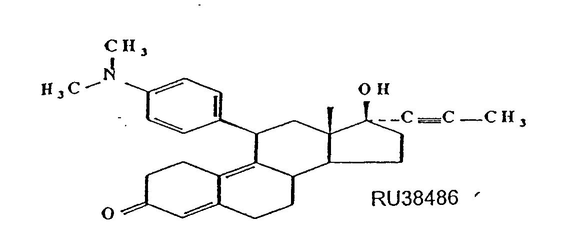

Als Glucocorticoid-Rezeptorantagonisten sind die in der nachfolgenden Formel angegebenen Verbindungen, die ebenfalls Gegenstand der vorliegenden Erfindung sind, aufgrund ihrer besonders guten Wirkung zur Behandlung vorgenannter Erkrankungen besonders geeignet: 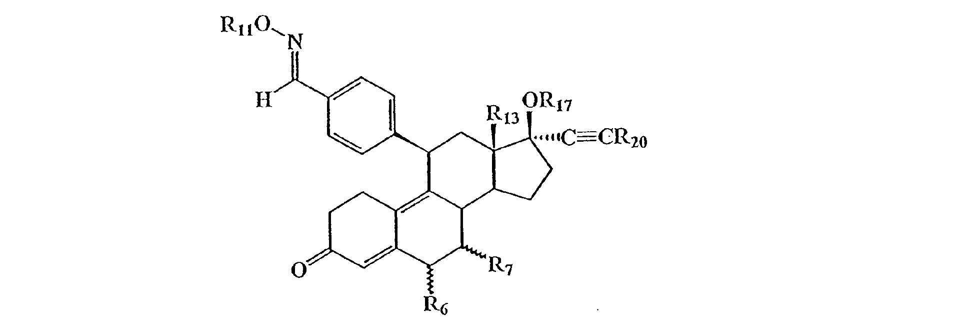 worin
R₆ ein Wasserstoffatom oder ein α-oder β-ständiges Halogenatom, wie Fluor, Chlor oder Brom, oder ein α- oder β-ständiger C₁₋₆ Alkylrest ist,
R₇ unabhängig von R₆ ein Wasserstoffatom oder ein α- oder β-ständiges Halogenatom, wie Fluor, Chlor oder Brom, oder ein α- oder β-ständiger C₁₋₆ Alkylrest ist,
R₁₁ ein Wasserstoffatom, ein C₁₋₆ Alkylrest, C₁₋₆ Acylrest, ein Rest CONHR₂₁, COSR₂₂ oder COOR₂₂ bedeutet, wobei R₂₁ einen C₁₋₆ Alkylrest oder einen unsubstituierten oder substituierten C₆-C₁₀-Arylrest und R₂₂ einen C₁₋₆ Alkylrest oder einen unsubstituierten oder substituierten C₆-C₁₀ Arylrest darstellt,
R₁₃ für einen Metyl- oder Ethylrest steht,
R₁₇ ein Wasserstoffatom oder ein C₁₋₆ Alkylrest oder C₁₋₆ Acylrest ist und
R₂₀ ein Wasserstoffatom, ein C₁₋₆ Alkylrest, ein unsubstituierter oder substituierter C₆₋₁₂ Arylrest ist.

Beispiele der Arylreste sind Phenyl- bis Naphthylreste. Diese können einen oder mehrere, voneinander unabhängig gewählte Substituenten aufweisen. Die Substituenten können sich in o-, m-und/oder p-Stellung befinden. Beispiele der Substituenten sind verzweigte oder geradhaltige C₁-C₆-Alkylreste, wie Methyl, Ethyl, Propyl oder Isopropyl, Halogene, wie Chlor oder Brom, und Pseudohalogene, wie Azid- oder Rhodonid-Gruppen. Die Substituenten der Arylreste, insbesondere bei R₂₀, können weiterhin substituierte Phenylreste sein ausgewählt unter Sulfonamide vom Typ C₆H₄SO₂NR₂₁R₂₂, Sulfonalkyle vom Typ C₆H₄SO₂R₂₃, Aminosulfone vom Typ C₆H₄NHSO₂R₂₃ oder Hydroxysulfonalkyle vom Typ C₆H₄OSO₂ R₂₄, wobei R₂₁ bis R₂₄ geradkettige oder verzweigte C₁-C₆-Alkyle, wie Methyl, Ethyl, Propyl oder Isopropyl, sind.

Aufgrund ihrer besonders positiven Eigenschaften zur Behandlung vorgenannter Erkrankungen bevorzugte Vertreter der erfindungsgemäßen Verbindungen sind:
4-(17α-Ethinyl-17β-hydroxy-3-oxoestra-4,9-dien-11β-yl)benzaldehyd-1(E)-oxim,
4-(17α-Ethinyl-17β-methoxy-3-oxoestra-4,9-dien-11β-yl)bendaldehyd-1(E)-oxim,
4-(17β-Hydroxy-17α-propinyl-3-oxoestra-4,9-dien-11β-yl)benzaldehyd-1(E)-oxim,
4-(17β-Methoxy-17a-propinyl-3-oxoestra-4,9-dien-1 β-yl)benzaldehyd-1(E)-oxim,
4-[17β-Hydroxy-17α,21-(phenyl)19nor-3-oxopregna-4,9-dien-20-yn-11β-yl]-benzaldehyd-1 (E)-oxim,
4-[17β-Hydroxy-17α,21-(4'-methylphenyl)19-nor-3-oxopregna-4,9-dien-20-yn-11β-yl]benzaldehyd-1(E)-oxim,
4-[17β-Hydroxy-17α,21-(tert.-butyl)19-nor-3-oxopregna-4,9-dien-20-yn-11β-yl]benzaldehyd-1(E)-oxim, 4-[17β-Hydroxy-17α,21-(4'-tert.-butylphenyl)19-nor-3-oxopregna-4,9-dien-20-yn-11β-yl]benzaldehyd-1 (E)-oxim
4-[17β-Hydroxy-17α,21-(4'-methylsulfonylphenyl)19nor-3-oxopregna-4,9-dien-20-yn-11β-yl]benzaldehyd-1 (E)-oxim,
4-[17β-Hydroxy-17α,21-(4'-methylsulfonylphenyl)19-nor-3-oxopregna-4,9-dien-20-yn-11β-yl]benzaldehyd-1(E)-[N-(ethylamin)carbonyl]oxim,
4-[17β-Hydroxy-17α,21-(4'-methylsulfonylphenyl)19-nor-3-oxopregna-4,9-dien-20-yn-11β-yl]benzaldehyd-1(E)-[S-(ethylthio)carbonyl]oxim,
4-[17β-Hydroxy-17a,21-(4'-methylsulfonylphenyl)19-nor-3-oxopregna-4,9-dien-20-yn-11β-yl]benzaldehyd-1(E)-[O-(ethyloxy)carbonyl]oxim,
4-(17β-Hydroxy-17α-propinyl-13β-ethyl-3-oxoestra-4,9-dien-1 1β-yl)benzaldehyd-1(E)-oxim,
4-(6ß-Chlor-17β-hydroxy-17α-propinyl-3-oxoestra-4,9-dien-11β-yl)benzaldehyd-1(E)-oxim,
4-(7a-Methyl-17β-hydroxy-17α-propinyl-3-oxoestra-4,9-dien-11β-yl)benzaldehyd-1 (E)-oxim,
4-(17β-Acetoxy-17α-propinyl-3-oxoestra-4,9-dien-11β-yl)benzaldehyd-1(E)-oxim,
4-[17ß-Acetoxy-17α,21-(4'-methylsulfonylphenyl)19-nor-3-oxopregna-4,9-dien-20-yn-11β-yl]benzaldehyd-1(E)-O-acetyloxim
4-(17β-Hydroxy-17a-propinyl-3-oxoestra-4,9-dien-11β-yl)benzaldehyd-1(E)-[N-trifluormethoxy phenylamino)carbonyl]oxim.

Vorstehende Verbindungen sind besonders gut zur Behandlung und/oder zur Vorbeugung von durch einen Androgen-Mangel bedingte Erkrankungen geeignet.

Die vorliegende Erfindung betrifft ferner ein Verfahren zur Herstellung der erfindungsgemäßen Verbindungen wobei 11β-Formylphenylsteroide der allgemeinen Formel 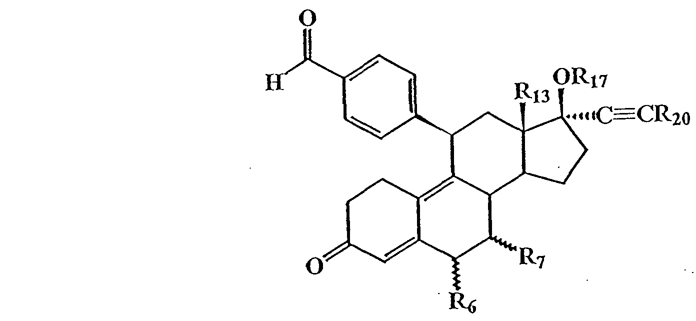 mit Hydroxylamin, Salzen von Hydroxylamin, wie Hydroxylaminhydrochlorid oder Hydroxylaminhydrogensulfat, in Lösungsmitteln, wie Pyridin, Dimethylformamid oder Alkoholen, in Gegenwart von Basen, wie Alkali-oder Erdalkalicarbonaten, -hydroxiden oder Kalium *tert.*-butanolat, zu den entsprechenden 11β-Benzaldoximen der vorgenannten Formel umgesetzt und Hydroxylgruppen gegebenfalls verestert oder verethert oder in eine Urethangruppe überführt werden.

Mit diesem erfindungsgemäßen Verfahren ist es in besonders einfacher, schneller und kostengünstiger Weise möglich, die erfindungsgemäßen Verbindungen in hoher Reinheit und mit hoher Ausbeute herzustellen.

Die folgenden Beispiele erläutem die Erfindung, ohne sie aber darauf einzuschränken.

### Beispiel 1: Erhöhung der Testosteron-Produktion durch Glucocorticoid Rezeptorantagonisten in Leydig-Zellen

### Methodenbeschreibung

Testikuläre Zellen wurden aus den Hoden von geschlechtsreifen, 12 Wochen alten männlichen Wistar-Ratten mittels Collagenasebehandlung präpariert. Leydig-Zellen wurden aus dieser Präparation durch kontinuierliche Percollgradientenzentrifugation gewonnen.

Frisch präparierte Leydigzellen wurden in Mikrotiterplatten in 100 µl DMEM/F12 mit Phenolrot (20 000 Zellen / well) mit Zusatz von 0,1 % BSA, Penicillin [100 U/ml] und Streptomycin [100µg/ml] eingesät. Nach einer Stunde erfolgte ein Mediumwechsel mit Substanzzugabe. Für 18 h wurden die Leydig-Zelle mit Dexamethason allein oder mit den Glucocorticoid-Rezeptorantagonisten gemeinsam inkubiert (250 µl DMEM Medium/well mit Zusatz von Penicillin [100 U/ml] und Streptomycin [100µg/ml]). Das DMEM Medium enthielt kein Phenolrot. Danach erfolgte ein erneuter Mediumwechsel und die Testosteronproduktion der Zellen wurde mit humanem Choriongonadotropin (hCG, Sigma, 0,5 ng/ml) für 8 h in Anwesenheit von Dexamethason bzw. den Glucocorticoid-Rezeptorantagonisten stimuliert. Als Lösungsmittel für Dexamethason und die Glucocorticoid-Rezeptorantagonisten wurde Ethanol eingesetzt; die Ethanol-Endkonzentration betrug maximal 0,2 %.

Aliquote des Überstandes wurden bei -70°C eingefroren. Testosteron wurde mit einem kommerziellen Testosteron-Elisa (IBL, Hamburg) gemessen. Die Standardkurve des Testosteron-Elisa wurde den Zellkulturbedingungen angepaßt.

Parallel zur Testosteronmessung erfolgte nach den 26 Stunden eine Bestimmung der Zellschädigung durch Messung der Aktivität der mitochondrialen Succinatdehydrogenase (WST-1 Test, Böhringer Mannheim).

Die Ergebnisse sind in der nachfolgenden Tabelle dargestellt.

### Ergebnisse

Die Stimulation von kultivierten Leydig-Zellen mit hCG resultiert in einem mehrfachen Anstieg der Testosteron-Produktion. Die Behandlung von Leydig-Zellen mit Dexamethason in einer Konzentration von 10⁻⁷ M führte zu einer signifikanter Verminderung der hCG-induzierten Testosteron-Produktion in vitro. Simultane Anwendung von Glucocorticoid-Rezeptorantagonisten in einer Dosis von 10⁻⁶ M verhindert vollständig die Hemmung der Testosteron-Produktion durch Dexamethason (Tabelle 1).

Die Aktivität der mitochondrialen Succinatdehydrogenase in kultivierten Leydig-Zellen wird durch eine Stimulation mit hCG in der angegebenen Dosis nicht verändert. Behandlung der Zellen mit Dexamethason in einer Dosis von 10⁻⁷ M führt zu signifikanter Hemmung der mitochondrialen Succinatdehydrogenase-Aktivität; die Letztere wird durch die simultane Gabe eines Glucocorticoid-Rezeptorantagonisten in einer Konzentration von 10⁻⁶ M vollständig aufgehoben (Tabelle 2).

### Schlußfolgerungen

Die Aktivierung des Glucocorticoid-Rezeptors in Leydig-Zellen resultiert in einer signifikanten Verminderung der endokrinen sekretorischen Aktivität (demonstriert durch emiedrigte Testosteron-Produktion nach hCG-Stimulation) sowie in deutlichen Symptomen einer Zellschädigung (angezeigt durch erniedrigte Aktivität der mitochondrialen Succinatdehydrogenase). Eine Behandlung mit Antagonisten des GR verhindert das Auftreten dieser Symptome, die für eine morphologische und funktionelle Schädigung charakteristisch sind und den Zustand eines Hypogonadismus weitgehend definieren.

**Tabelle 1:**

| | | | |
|---|---|---|---|
| Durch hCG stimulierte Testosteronproduktion von primären Rattenleydigzellen nach Behandlung mit 100 nM Dexamethason [DEX] allein oder bei zusätzlicher Gabe von Glucocorticoid-Rezeptorantagonisten [1 uM]. Die Daten repräsentieren Mittelwerte ± Standardabweichungen (SD) aus 6-12 Parallelbestimmungen; Sterne bezeichnen signifikante Differenzen zur Versuchsbedingung hCG allein | | | |

| Versuchsbedingung | Testosteron-Produktion (ng/ml) | | |
|---|---|---|---|
| | Mittelwert ± SD (ng/ml) | n | Mittelwert ± SD (%) |
| Basal | 0,17 ± 0,03 | 6 | 0,00 ± 0,63 |
| hCG allein [0.5 ng/ml] | 4,26 ± 1,11 | 12 | 100,00 ± 27,25 |
| hCG[0.5 ng/ml] + DEX | 2,00 ± 0,58 P | 12 | 44,63 ± 14,08 P |
| hCG + DEX + RU38486 | 4,48 ± 1,14 | 6 | 105,23 ± 27,83 |
| hCG + DEX + GRA1 | 3,57 ± 0,83 | 6 | 83,00 ± 20,40 |
| hCG + DEX + GRA2 | 3,48 ± 0,64 | 6 | 80,97 ± 15,47 |
| hCG + DEX + GRA3 | 4,52 ± 1,01 | 6 | 106,30 ± 24,75 |

Ru 38 486 = 11β-Dimethylaminophenyl-17β-hydroxy-17α-propinylestra,4,9-dien-3-on
GRA 1 = 4-(17β-Methoxy-17α-(methoxymethyl)-estra-4,9-dien-3-on-11β-yl]2,2,2- trifluoracetophenon-1 (Z)-oxim
GRA 2 = 11β-{5-(1,3-Benzodioxol)-17α,21-[(4'-methylsulfonyl)phenyl]17β-hydroxy-19norpregna-4,9-dien-20-yn-3-on
GRA 3 = 4-[17β-Hydroxy-17α,21-(4'-methylsulfonylphenyl)19-nor-3-oxopregna-4,9dien-20-yn-11 β-yl]benzaldehyd-1(E)-oxim

**Tabelle 2:**

| | | |
|---|---|---|
| Aktivität der mitochondrialen Succinatdehydrogenase in primären Leydig-Zellkulturen nach Behandlung mit 100 nM Dexamethason [DEX] allein oder zusätzlicher Gabe von Glucocorticoid-Rezeptorantagonisten [1 µM]. Die Daten sind als Mittelwerte ± Standardabweichung (SD) aus 5-12 Parallelbestimmungen; Sterne bezeichnen signifikante Unterschiede zur Versuchsbedingung hCG allein. | | |

| Succinatdehydrogenase-Aktivität (Absorptionseinheiten) | | |
|---|---|---|
| Versuchsbedingung | Mittelwert ± SD | n |
| Basal | 0,269 ± 0,020 | 6 |
| hCG allein [0.5 ng/ml] | 0,281 ± 0,041 | 12 |
| hCG[0.5 ng/ml] + DEX | 0,192 ± 0,036 * | 12 |
| hCG + DEX + RU38486 | 0,311 ± 0,031 | 6 |
| hCG + DEX + GRA1 | 0,289 ± 0,040 | 5 |
| hCG + DEX + GRA2 | 0,363 ± 0,032 | 6 |
| hCG + DEX + GRA3 | 0,313 ± 0,018 | 6 |

### Beispiel 2: Erhöhung der Testosteron-Produktion durch Verabreichung von Glucocorticoid- Rezeptorantagonisten in Ratten

### Methodenbeschreibung

Geschlechtsreife, drei Monate alte, männliche Ratten der CD-Zuchtlinie (Charles River GmbH, Sulzfeld, Deutschland) wurden in Gruppen zu 4 Tieren unter kontrollierter Beleuchtung (Licht:Dunkelheit 12:12 Stunden) und freiem Zugang zu Nahrung und Trinkwasser gehalten. Nach einwöchiger Adaptation wurden die Tiere durch Exposition zu ovariektomierten östrogen-behandelten Weibchen auf das Vorhandensein eines männlichen Sexualverhaltens getestet (G. Dörner, Hormones and brain differentiation, Elsevier, Amsterdam, 1976, pp. 128-132). Nur Männchen, die an drei aufeinanderfolgenden Tagen ein robustes positives Verhalten (mehr als 5 komplette Sprünge innerhalb einer Beobachtungszeit von 5 Minuten) zeigten, wurden in die Testung aufgenommen. Die Tiere der Referenzgruppe erhielten täglich intraperitoneale Injektionen mit Dexamethason (Fortecortin; Merck, Darmstadt, Deutschland) nach dem folgenden Schema: 0,5 mg/kg am Versuchstag 1 bis 3, 1 mg/kg am Tag 4 bis 6, und 1,5 mg/kg am Tag 7 bis 9. Die Kontrolltiere wurden mit Placebo (0,9 %-ige sterile NaCI-Lösung) injiziert. Die Verum-Gruppe erhielt zusätzlich zur Dexamethason-Behandlung nach dem o.g. Schema tägliche subkutane Injektionen mit dem Glucocorticoidrezeptor-Antagonisten RU 38486 (Sigma, Deisenhofen, Deutschland) in Sesamöl-Lösung in einer Dosis von 10 mg/kg.

Männliches Sexualverhalten wurde nach der o.g. Methode am 9. Behandlungstag bewertet. Nach dem Verhaltenstest wurde Blut aus dem Retroorbitalplexus zur Bestimmung der Serum-Testosteronspiegel entnommen. Die Testosteronbestimmung erfolgte mit einem kommerziellen Test-Ansatz (IBL, Hamburg, Deutschland).

### Ergebnisse

Chronische Behandlung mit Dexamethason führte zu einer signifikanten Hemmung der männlichen Sexualaktivität im Vergleich zur Placebo-Gruppe. Gleichzeitige Verabreichung von Dexamethason und dem Glucocorticoid-Rezeptorantagonisten RU 38486 resultierte in einer Beibehaltung des männlichen Sexualverhaltens auf dem Niveau der Placebo-Gruppe.

Tägliche Injektionen von Dexamthason führten zu einer signifikanten Erniedrigung der Serum-Testosteronspiegel im Vergleich zu Placebo-behandelten Tieren. Die simultane Verabreichung des Glucocorticoid-Rezeptorantagonisten RU 38486 verhinderte die Erniedrigung der Serum-Testosteronkonzentrationen.

### Schlußfolgerungen

Chronische Behandlung mit steigenden Dosen von Glucocorticoiden stellt ein reliables pathophysiologisches Modell der exzessiven Aktivierung des Glucocorticoidrezeptors dar. Zu den pathologischen Manifestationen dieses Zustandes gehören die beobachtete Hemmung des männlichen Sexualverhaltens und die Emiedrigung der endokrinen sekretorischen Aktivität der männlichen Gonaden. Diese Symptome sind u.a. für den Hypogonadismus charakteristisch und werden auch bei anderen Krankheitsbildem beobachtet, die mit erhöhter Glucocorticoid-Sekretion einhergehen (z.B. Cushing-Syndrom, chronischer Stress).

Behandlung mit einem bekannten Antagonisten des Glucocorticoid-Rezeptors verhindert die durch Dexamethason verursachte Hemmung der gonadalen endokrinen Sekretion bzw. der männlichen Sexualaktivität.

**Tabelle 3:**

| | | |
|---|---|---|
| Beeinflussung des männlichen Sexualverhaltens durch chronische Behandlung mit Dexamethason bzw. einer Kombination aus Dexamethason und dem Glucocorticoid-Rezeptorantagonisten RU 38486. Die Daten repräsentieren Mittelwert ± Standardfehler; die einzelnen Behandlungsgruppen bestehen aus 13-15 Tieren; Sterne bezeichnen signifikante Unterschiede zu Placebo-Behandlung | | |

| Behandlungsgruppe | Männliches Sexualverhalten (Sprünge in 5 Min.) | Serum-Testosteron (ng/ml) |
|---|---|---|
| Placebo | 10,2 ± 1,3 | 17,2 ± 2,3 |
| Dexamethason | 4,0 ± 0,9 * | 2,3 ± 0,2 * |
| Dexamethason + RU38486 | 9,2 ± 1,0 | 20,2 ± 4,9 |

### Beispiel 3: Herstellung von 4-(17α-Ethinyl-17β-methoxy-3-oxoestra-4,9-dien-11β-yl) benzaldehyd-1(E)-oxim

500 mg 4-(17α-Ethinyl-17β-methoxy-3-oxoestra-4,9-dien-11β-yl)benzaldehyd wurden unter Argon in 20 ml Pyridin mit 103 mg Hydroxylaminhydrochlorid bei Raumtemperatur umgesetzt. Nach 4 Stunden wurde der Ansatz in 600 ml Eiswasser eingerührt, wobei das Produkt weiß ausflockt. Es wird abgesaugt und an der Luft getrocknet. Es wurden 489 mg hellgelben Schaum erhalten. Die Reinigung des Rohproduktes erfolgte mittels präparativer Schichtchromatografie mit einem Toluol/Aceton-Gradien. Das dabei erhaltene Produkt wurde aus Aceton umkristallisiert. Es wurden 271 mg farblose Kristalle erhalten.
Schmelzpunkt: 149 bis 157 °C (Aceton)
α_{D}= +162 °C (Chloroform)
¹H-NMR (300 MHz, CDCl₃, TMS): 0.52 (s, 3H, H-18), 2.66 (s, 1H, C≡CH), 3.38 (s, 3H, OCH₃), 4.42 (d, 1H, J = 7.2 Hz, H-11), 5.80 (s, 1H, H-4), 7.20 (d, 2H, J = 8.1 Hz, arom. CH), 7.49 (d, 2H, J = 8.4 Hz, arom. CH), 8.11 (s.1H, CH=N), 8.39 (s,1H. OH).

### Herstellung der Ausgangsverbindung

### Stufe A

### 4-(3,3-Dimethoxy-5α,17β-dihydroxy-17a-ethinyl-estr-9-en-11β-yl)benzaldehyd-ethylenacetal

4,82 g 4-(3,3-Dimethoxy-5α-hydroxy-17oxoestr-9-en-11β-yl)benzaldehyd-ethylenacetal werden in 20 ml THF(abs) werden auf - 50 °C gekühlt. Man tropft 150 ml einer 0,5 M Ethinylmagnesiumbromidlösung in THF zu und läßt die Mischung auf Raumtemperatur kommen.

Nach 4 Stunden werden unter Kühlung 100 ml Ammoniumchloridlösung (10% ig) zugetropft, die Mischung wird mit Essigester extrahiert und wie üblich aufgearbeitet. Es werden 5,68 g Rohprodukt isoliert, das durch Chromatograpfie gereinigt wird. Man erhält einen farblosen Schaum, der direkt weiter verarbeitet wird.
α_{D}= - 27 °C (Chloroform)
¹H-NMR (300 MHz, CDCl₃, TMS): 0.42 (s, 3H, H-18), 2.58 (s, 1H, C≡CH), 3.21 und 3.22 (2s, je 3H, OCH₃), 4.03 - 4.15 (m, 4H, Ethylenketal), 4.32 (d, 1H, J = 7.2 Hz, H-11), 4.67 (s, 1H, OH), 5.75 (s, 1H, CH-acetal), 7.23 (d, 2H, J = 8.1 Hz, arom. CH), 7.38 (d, 2H, J = 8.1 Hz, arom. CH).

### Stufe B

### 4-(3,3-Dimethoxy-17a-ethinyl-5a-hydroxy-17β-methoxy-estr-9-en-11β-yl)benzaldehyd-ethylenacetal

1,01 g 4-(3,3-Dimethoxy-5α,17β-dihydroxy-17a-ethinyl-estr-9-en-11β-yl)benzaldehyd-ethylenacetal werden in 40 ml THF (abs) unter Argon bei einer Temperatur von -38°C nacheinander mit 2,2 ml einer 2,7 M Butyllithium-Lösung in THF und anschließend 0,86 ml Methyljodid versetzt. Man läßt auf Raumtemperatur kommen und zersetzt nach 30 Stunden durch Zugabe von Wasser. Es wird mit Essigester extrahiert und wie üblich aufgearbeitet. Man erhält 930 mg 4-(3,3-Dimethoxy-17α-ethinyl-5a-hydroxy-17ß-methoxy-estr-9-en-11ß-yl)benzaldehyd-ethylenacetal als braunen Schaum, der ohne Reinigung in die Stufe C eingesetzt wird.

¹H-NMR (300 MHz, CDCl₃, TMS): 0.42 (s, 3H, H-18), 2.58 (s, 1H, C≡CH), 3.21 und 3.22 (2s, je 3H, OCH₃), 3.38 (s, 1H, OCH₃), 4.02 - 4.16 (m, 4H, Ethylenketal), 4.32 (d, 1H, J = 7.2 Hz, H-11), 4.67 (s, 1H, OH), 5.75 (s, 1H, CH-acetal), 7.24 (d, 2H, J = 8.1 Hz, arom. CH), 7.37 (d, 2H, J = 8.1 Hz, arom. CH).

### Stufe C

### 4-(17α-Ethinyl-17β-methoxy-3-oxoestr-4,9-dien-11β-yl)benzaldehyd

417 mg 4-(3,3-Dimethoxy-17α-ethinyl-5a-hydroxy-17β-methoxy-estr-9-en-11β-yl)benzalde-hydethylenacetal werden in 20 ml Aceton gelöst, mit 2 ml Wasser und 200 mg p-Toluolsulfonsäure versetzt und 3 Stunden bei Raumtemperatur gerührt. Durch Zugabe von Eiswasser wird ein Rohprodukt gefällt, das abgesaugt, mit Wasser gewaschen und getrocknet wird. Das Rohprodukt (300 mg) wird durch Chromatografie gereinigt und umkristallisiert.
Schmelzpunkt: 197 bis 202 °C (CH₂Cl₂ / Aceton)
α_{D}= +132 °C (Chloroform)
¹H-NMR (300 MHz, CDCl₃, TMS): 0.49 (s, 3H, H-18), 2.67 (s, 1H, C≡CH), 3.38 (s, 3H, OCH₃), 4.46 (d, 1H, J = 7.2 Hz, H-11), 5.80 (s, 1H, H-4), 7.38 (d, 2H, J = 8.1 Hz, arom. CH), 7.81 (d, 2H, J = 8.4 Hz, arom. CH), 9.98 (s,1H, CH=O).

### Beispiel 4: Herstellung von 4-(17α-Ethinyl-17β-hydroxy-3-oxoestra-4,9-dien-11β-yl)benzaldehyd-1(E)-oxim

Herstellung analog Beispiel 3 aus 4-(17α-Ethinyl-17β-hydroxy-3-oxoestra-4,9-dien-11β-yl)benzaldehyd.
Schmelzpunkt: 219 bis 221 °C (tert.-Butylmethylether/n-Hexan)
α_{D}= +157 °C (Chloroform)
¹H-NMR (300 MHz, CDCl₃, TMS): 0.52 (s, 3H, H-18), 2.51 (s, 1H, OH), 2.65 (s, 1H, C≡CH), 4.44 (d, 1H, J = 7.2 Hz, H-11), 5.80 (s, 1H, H-4), 7.20 (d, 2H, J = 8.1 Hz, arom. CH), 7.59 (d, 2H, J = 8.4 Hz, arom. CH), 8.11 (s, 1H, CH=N), 8.64 (s,1H, OH).

Herstellung der Ausgangsverbindung 4-(17α-Ehtinyl-17β-hydroxy-3-oxoestra-4,9-dien-11β-yl)benzaldehyd
Zu 1,2 g 4-(3,3-Dimethoxy-17α-ethinyl-5a-hydroxy-17β-methoxy-estr-9-en-11β-yl)benzaldehydethylenacetal in 20 ml Aceton werden 2 ml Wasser zugefügt. Nach Zugabe von 300 mg 4-Toluolsulfonsäure wird die Mischung bei Raumtemperatur 2 Stunden gerührt. Die Reaktionslösung wird in 300 ml Eiswasser eingerührt, wobei ein flockiger Niederschlag ausfällt. Man neutralisiert mit NaHCO₃-Lösung und rührt 30 Minuten nach, saugt den Niederschlag ab, wäscht mit Wasser und trocknet unter Vakuum. Man erhält 786 mg als gelben Schaum. Das Rohprodukt wird durch präparative Schichtchromatografie an Kieselgel PF_{254+366 nm} (MERCK AG) mit dem Laufmittel Toluen/Aceton 4:1 gereinigt. Man erhält 430 mg an 4-(17α-Ethinyl-17β-hydroxy-3-oxoestra-4,9-dien-11β-yl)benzaldehyd. Die Umkristallisation erfolgt aus tert.Butylmethylether unter Zugabe von n- Hexan in der Siedehitze.
Schmelzpunkt: 190 bis193 °C (tert.-Butylmethylether/n-Hexan)
α_{D} = + 150° (CHCl₃)
¹H-NMR: [300 MHz, CDCl_{3,} TMS] (δ, ppm): 0.50 (s, 3H, H-18); 2.29 (s, 1H, OH), 2.65 (s, 1H, C≡CH), 4.49 (d, 1H, J = 6.9 Hz, H-11α); 5.81 (s, 1H, H-4); 7.38 (d, 2H, J = 8,1 Hz, H-2'), 7.81 (d, 2H, J = 8.4 Hz, H-3'); 9.98 (s, 1H, CH=O).

### Beispiel 5: Herstellung von 4-(17β-Hydroxy-17α-propinyl-3-oxoestra-4,9-dien-11β-yl)benzaldehyd-1 (E)-oxim

Herstellung gemäß Beispiel 3 aus 4-(17β-Hydroxy-17α-propinyl 3-oxoestra-4,9-dien-11β-yl)benzaldehyd
Schmelzpunkt: 149 bis 151 °C (Aceton / n-Hexan)
α_{D} = + 140° (CHCl₃)
¹H-NMR: [300 MHz, CDCl₃, TMS] (8, ppm): 0.49 (s, 3H, H-18), 1.90 (s, 3H, propinyl), 4.43 (d, 1H, J = 7.2 Hz, H-11α), 5.80 (s, 1H, H-4), 7.19 (d, 2H, J = 8.4 Hz, H-2'), 7.48 (d, 2H, J = 8.4 Hz, H-3'), 8.11 (s,1H, CH=N), 8.81 (s,1H NOH).
HPLC: 98 % F bei 264 nm

### Beispiel 6: Herstellung von 4-(17β-Methoxy-17α-propinyl-3-oxoestra-4,9-dien-11β-yl)benzaldehyd-1(E)-oxim

Herstellung erfolgt gemäß Beispiel 3 aus 4-(17β-Hydroxy-17α-propinyl 3-oxoestra-4,9-dien-11β-yl)benzaldehyd.
farbloser Schaum
α_{D} = + 143° (CHCl₃)
¹H-NMR: [300 MHz, CDCl₃, TMS] (δ, ppm): 0.47 (s, 3H, H-18), 1.90 (s, 3H, propinyl), 3.38 (s, 3H. OCH₃), 4.44 (d, 1H, J = 7.2 Hz, H-11α), 5.80 (s, 1H, H-4), 7.19 (d, 2H, J = 8.4 Hz, H-2'), 7.48 (d, 2H, J = 8.4 Hz, H-3'), 8.11 (s,1H, CH=N), 8.81 (s,1H, NOH).

### Beispiel 7: Herstellung von 4-(17β-Hydroxy-17α-propinyl-3-oxoestra-4,9-dien-11β-yl)benzaldehyd-1(E)-{N-[(trifluormethoxy)phenylamino]carbonyl}oxim

725 mg 4-(17β-Hydroxy-17α-propinyl 3-oxoestra-4,9-dien-11β-yl)benzaldehyd-1(E)-oxim werden unter Argon in 15 ml Toluen suspendiert. Man fügt 0,8 ml 4-Trifluormethoxyphenylisocyanat zu und rührt 2 Stunden bei 50 °C. Es wird abgekühlt, 30ml wässriger Ammoniak zugegeben, mit Dichlormethan extrahiert, die organische Phase neutral gewaschen, über Natriumsulfat getrocknet, abfiltriert und unter Vakuum eingeengt. Das Rohprodukt (1,3 g) wird durch Chromatografie gereinigt. Man erhält 480 mg 4-(17β-Hydroxy-17α-propinyl-3-oxoestra-4,9-dien-11β-yl)benzaldehyd-1(E)-{N-[(trifluormethoxy)-phenylamino]carbonyl}oxim als farblosen Schaum.
α_{D} = + 132° (CHCl₃)
¹H-NMR: [400 MHz, CDCl₃, TMS] (δ, ppm): 0.51 (s, 3H, H-18), 1.66 (s, 1H, OH), 1.92 (s, 3H, propinyl), 4.49 (d, 1H, J = 7.2 Hz, H-11α), 5.81 (s, 1H, H-4), 7.22 (d, 2H, J = 9.2 Hz, arom. CH), 7.29 (d, 2 H, J = 14.9 Hz, arom. CH), 7.56 (d, 2H, J = 12.0 Hz, arom.CH), 7.65 (d, 2H, J = 8.0 Hz, arom. CH), 8.19 (s,1H, NH), 8.40 (s,1H, CH=N).

### Beispiel 8: Herstellung von 4-[17β-Hydroxy-17a,21-(phenyl)19-nor-3-oxopregna-4,9-dien-20-yn-11 β-yl]benzaldehyd-1(E)-oxim

Herstellung erfolgte gemäß Beispiel 3 aus 4-[17β-Hydroxy-17a,21-(phenyl)19-nor-3-oxopregna-4,9-dien-20-yn-1 1β-yl]benzaldehyd.
Herstellung der Ausgangsverbindung
1,93 g 4-(3,3-Dimethoxy-5a-hydroxy-17-oxoestr-9-en-11β-yl)benzaldehyd-ethylenacetal werden nach Beispiel 3 Stufe A mit 30 ml einer 1M Phenylethinylmagnesiumbromidlösung in THF umgesetzt. Das Rohprodukt 4-[3,3-Dimethoxy-5α,17β-dihydroxy-17α-(phenylethinyl)-estr-9-en-11β-yl]benzaldehydethylenacetal (3,5 g) wird durch Säulenchromatographie an Kieselgel 60 (0,04 -0,063 mm) mit einem Toluol/Aceton-Gradienten gereinigt und anschließend in 20 ml Aceton mit 200 mg p-Toluolsulfonsäure bei Raumtemperatur hydrolysiert. Man rührt in Eiswasser ein, saugt den Niederschlag ab, wäscht neutral und trocknet. Der 4-[17ß-Hydroxy-17a,21-(phenyl)19-nor-3-oxopregna-4,9-dien-20-yn-11β-yl]benzaldehyd wird als farbloser Schaum isoliert, der direkt in die Stufe der Oximierung eingesetzt wird.

Schmelzpunkt: ab 137 °C (Zersetzung, Aceton /tert.-Butylmethylether)
α_{D} = + 143° (CHCl₃)
¹H-NMR: [400 MHz, CDCl₃, TMS] (δ, ppm): 0.57 (s, 3H, H-18), 3.38 (s, 3H. OCH₃), 4.45 (d, 1H, J = 7.2 Hz, H-11α), 5.79 (s, 1H, H-4), 7.20 (d, 2H, J = 8.0 Hz, H-2'), 7.48 (d, 2H, J = 8.4 Hz, H-3'), 7.3-7.46 (m, 5H, Phenyl), 8.05 (breites s, 1H NOH), 8.11 (s, 1H, CH=N).
LC/MS: 98,8 % F bei M⁺ + 1 = 492

### Beispiel 9: Herstellung von 4-[17β-Hydroxy-17α,21-(4'-methylsulfonylphenyl)19-nor-3-oxopregna-4,9-dien-20-yn-11β-yl]benzaldehyd-1(E)-oxim

Herstellung erfolgte gemäß Beispiel 3 aus 4-[17ß-Hydroxy-17a,21-(4'-methylsulfonylphenyl)19-nor-3-oxopregna-4,9-dien-20-yn-1 1β-yl]benzaldehyd.

Schmelzpunkt: ab 180 °C (Zersetzung, Dichlormethan /tert.-Butylmethylether)
α_{D} = - 5° (CHCl₃)
¹H-NMR: [400 MHz, CDCl₃, TMS] (δ, ppm): 0.58 (s, 3H, H-18), 2.41 (s,1H, OH), 3.05 (s, 3H, SO₂CH₃), 4.45 (breites s, 1H, H-11α), 5.79 (s, 1H, H-4), 7.20 (d, 2H, J = 8.0 Hz, H-2'), 7.49 (d, 2H, J = 8.0 Hz, H-3'), 7.62 (d, 1H, J = 12.0 Hz, arom. CH), 7.90 (s, 2H, J = 12,4 Hz, arom. CH), 5H, Phenyl), 8.03 (s, 1H, NOH), 8.10 (s, 1H, CH=N).
LC/MS: 97,6 % F bei M⁺ + 1 = 570
Herstellung der Ausgangsverbindung
4-[17ß-Hydroxy-17α,21-(4'-methylsulfonylphenyl)19-nor-3-oxopregna-4,9-dien-20-yn-11β-yl]benzaldehyd.

Unter Argon werden 14,8 mg Palladiumacetat und 34,5 mg Triphenylphosphin in 5 ml Triethylamin suspendiert. Man rührt 10 Minuten nach und fügt 25 mg Kupferjodid und 309,2 mg 4-Bromphenylmethylsulfon fest zu. Zu der grünlich gefärbten Lösung werden 527 mg 4-(17α-Ethinyl-17β-hydroxy-3-oxoestra-4,9-dien-11β-yl-)benzaldehyd in 20 ml abs. THF langsam addiert, wobei ein Farbwechsel erfolgt. Nach 12 Stunden wird die Lösung in Eiswasser eingerührt, der dabei ausfallende Niederschlag wird abgesaugt, neutral gewaschen und getrocknet. Das Rohprodukt an 4-[17β-Hydroxy-17α,21-(4'-methylsulfonylphenyl)19-nor-3-oxopregna-4,9-dien-20-yn-1 1 ß-yl]benzaldehyd (715 mg) wird durch präparative Schichtchromatografie an Kieselgel 60 PF₂₅₄ mit einem Gemisch aus Toluol/Essigester/tert.-Butylmethylether gereinigt. Schmelzpunkt: 235 bis 237 °C (Aceton)
α_{D} = - 21° (CHCl₃)
¹H-NMR: [400 MHz, CDCl₃, TMS] (δ, ppm): 0.56 (s, 3H, H-18), 3.06 (s, 3H, SO₂CH₃), 4.52 (s, 1H, J = 7.2 Hz, H-11α), 5.80 (s, 1 H, H-4), 7.38 (d, 2H, J = 8.0 Hz, H-2'), 7.63 (d, 2H, J = 8.4 Hz, H-3'), 7.82 (d, 1H, J = 8.8 Hz, arom. CH), 7.91 (s, 2H, J = 8.4 Hz, arom. CH), 9.97 (s, 1H, CH=O).
LC/MS: 98,0 % F bei M⁺ + 1 = 555
HPLC: 98 % F bei 262 nm

### Beispiel 10: Herstellung von 4-[17β-Hydroxy-17α,21-(4'-methylsulfonylphenyl)19-nor-3-oxopregna-4,9-dien-20-yn-11β-yl]benzaldehyd-1(E)-[N-(ethylamino)carbonyl]oxim

Herstellung erfolgte gemäß Beispiel 7 durch Umsetzung von 4-[17β-Hydroxy-17α,21-(4'methylsulfonylphenyl)19-nor-3-oxopregna-4,9-dien-20-yn-11β-yl]benzaldehyd-1(E)-[N-(ethylamino)carbonyl]oxim mit Ethylisocyanat in Toluol.
Schmelzpunkt: 158 bis 162 °C (Toluol/ Aceton / n-Hexan)
α_{D} = - 2 ° (CHCl₃)
¹H-NMR: [400 MHz, CDCl₃, TMS] (δ, ppm): 0.58 (s, 3H, H-18), 1.23 (t, 3H, CH₂CH₃), 2.51 (s, 1H, OH), 3.06 (s, 3H, SO₂CH₃), 3.38 (m, 2H, CH₂CH₃), 4.48 (d, 1H, J = 4.8 Hz, H-11α), 5.79 (s, 1H, H-4), 6.23 (t, 1H, NH), 7.28 (d, 2H, J = 9.2 Hz, H-2'), 7.59 (d, 2H, J = 8.4 Hz, H-3'), 7.62 (d, 1H, J = 8.4 Hz, arom. CH), 7.89 (s, 2H, J = 8.8 Hz, arom. CH), 8.29 (s, 1H, CH=NOR). LC/MS: 96,6 % F bei M⁺ + 1 = 641

### Beispiel 11: Herstellung von 4-(17β-Hydroxy-17α-propinyl-3-oxoestra-4,9-dien-11β-yl)benzaldehyd-1(E)-[S-(ethylthio)carbonyl]oxim

450 mg 4-(17β-Hydroxy-17a-propinyl 3-oxoestra-4,9-dien-11β-yl)benzaldehyd-1(E)-oxim werden in 10 ml Pyridin bei Raumtemperatur mit 1,5 ml Chlorkohlensäureetiolester versetzt, 2 Stunden gerührt und in Eiswasser eingegossen. Man saugt ab, wäscht neutral, trocknet und reinigt durch Chromatografie. Man erhält 210 mg 4-(17β-Hydroxy-17α-propinyl-3-oxoestra-4,9-dien-11β-yl)benzaldehyd-1(E)-[S-(ethylthio)carbonyl]oxim als farblosen Schaum.
α_{D} = + 139° (CHCl₃)
¹H-NMR: [400 MHz, CDCl₃, TMS] (δ, ppm): 0.50 (s, 3H, H-18), 1.21 (t, 3H, CH₂CH₃), 1.67 (s, 1H, OH), 1.92 (s, 3H, propinyl), 3.37 (m, 2H, CH₂CH₃), 4.47 (d, 1H, J = 7.2 Hz, H-11α), 5.80 (s, 1H, H-4), 7.22 (d, 2H, J = 8.4 Hz, arom. CH), 7.55 (d, 2H, J = 8.8 Hz, arom.CH), 8.39 (s,1H, CH=N).

### Beispiel 12: Herstellung von 4-(17ß-Hydroxy-17a-propinyl-3-oxoestra-4,9-dien-11β-yl)benzaldehyd-1(E)-O-acetyloxim

300 mg 4-(17β-Hydroxy-17α-propinyl 3-oxoestra-4,9-dien-11β-yl)benzaldehyd-1(E)-oxim werden in 10 ml Pyridin bei Raumtemperatur mit 1,0 ml Acetanhydrid versetzt, 2 Stunden gerührt und in Eiswasser eingegossen. Man saugt ab, wäscht neutral, trocknet und reinigt durch Chromatografie. Man erhält 125 mg 4-(17β-Hydroxy-17α-propinyl-3-oxoestra-4,9-dien-11β-yl)benzaldehyd-1(E)-Oacetyloxim als farblosen Schaum.
α_{D} = + 133° (CHCl₃)
¹H-NMR: [400 MHz, CDCl₃, TMS] (δ, ppm): 0.52 (s, 3H, H-18), 1.64 (s, 1H, OH), 1.92 (s, 3H, propinyl), 2.12 (s, 3H, OCOCH₃), 4.45 (d, 1H, J = 7.2 Hz, H-11α), 5.80 (s, 1H, H-4), 7.21 (d, 2H, J = 8.4 Hz, arom. CH), 7.56 (d, 2H, J = 8.4 Hz, arom.CH), 8.38 (s,1H, CH=N).

## Patentansprüche

1. Verwendung von Glucocorticoid-Rezeptorantagonisten zur Vorbeugung und/oder Behandlung von durch verminderte Androgen-Produktion verursachte Erkrankungen.

2. Verwendung nach einem der vorhergehenden Ansprüche, wobei die Erkrankung ausgewählt ist aus Hypogonadismus beim Mann, sexuelle Dysfunktion beim Mann und Infertilität.

3. Verwendung nach einem der vorhergehenden Ansprüche, wobei der Glucocorticoid-Rezeptorantagonist die Typ-1-Transkriptionsinduktion der Glucocorticoid-Rezeptor-Gene antagonisiert.

4. Verwendung nach einem der vorhergehenden Ansprüche, wobei der Glucocorticoid-Rezeptorantagonist die Typ-2-Transkriptionsinhibierung im wesentlichen nicht antagonisiert.

5. Verwendung nach einem der vorhergehenden Ansprüche, wobei der Glucocorticoid-Rezeptorantagonist im wesentlichen nicht an andere Steroid-Rezeptoren bindet.

6. Verwendung nach einem der vorhergehenden Ansprüche, wobei der Glucocorticoid-Rezeptorantagonist die durch Glucocorticoid-Rezeptoren vermittelten Signale nicht vollständig hemmt.

7. Verwendung nach einem der vorhergehenden Ansprüche, wobei das Androgen Testosteron ist.

8. Verbindung der Formel 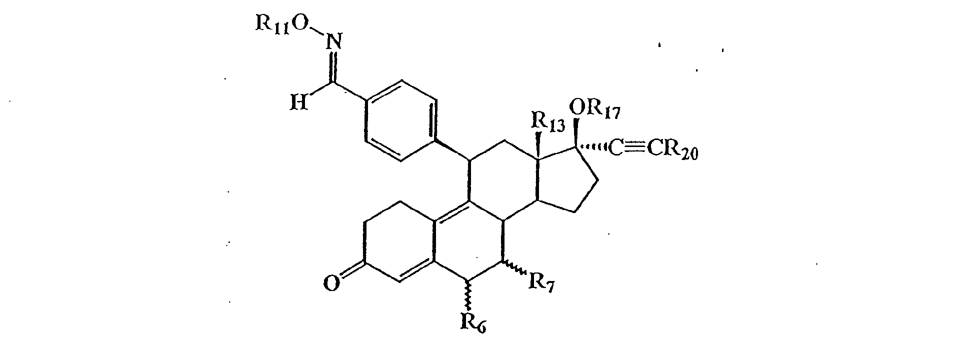 worin
R₆ ein Wasserstoffatom oder ein α-oder β-ständiges Halogenatom oder ein α- oder β-ständiger C₁₋₆ Alkylrest ist,
R₇ unabhängig von R₆ ein Wasserstoffatom oder ein α- oder β-ständiges Halogenatom oder ein α- oder β-ständiger C₁₋₆ Alkylrest ist,
R₁₁ ein Wasserstoffatom, ein C₁₋₆ Alkylrest, C₁₋₆ Acylrest, ein Rest CONHR₂₁, COSR₂₂ oder COOR₂₂ bedeutet, wobei R₂₁ einen C₁₋₆ Alkylrest oder einen unsubstituierten oder substituierten C₆-C₁₀-Arylrest und R₂₂ einen C₁₋₆ Alkylrest oder einen Arylrest darstellt,
R₁₃ für einen Metyl- oder Ethylrest steht,
R₁₇ ein Wasserstoffatom oder ein C₁₋₆ Alkylrest oder C₁₋₆ Acylrest ist und
R₂₀ ein Wasserstoffatom, ein C₁₋₆ Alkylrest, ein unsubstituierter oder substituierter C₆₋₁₂ Arylrest ist.

9. Verbindung nach Anspruch 8, wobei sie ausgewählt ist aus
4-(17α-Ethinyl-17β-hydroxy-3-oxoestra-4,9-dien-11,8-yl)benzaldehyd-1 (E)-oxim,
4-(17a-Ethinyl-17β-methoxy-3-oxoestra-4,9-dien-11β-yl)bendaldehyd-1(E)-oxim,
4-(17β-Hydroxy-17a-propinyl-3-oxoestra-4,9-dien-11β-yl)benzaldehyd-1(E)-oxim
4-(17β-Methoxy-17a-propinyl-3-oxoestra-4,9-dien-11β-yl)benzaldehyd-1(E)-oxim,
4-[17β-Hydroxy-17a,21 -(phenyl)19nor-3-oxopregna-4,9-dien-20-yu-11β-yl]-benzaldehyd-1(E)-oxim,
4-[17β-Hydroxy-17α,21-(4'-methylphenyl)19-nor-3-oxopregna-4,9-dien-20-yn-11β-yl]benzaldehyd-1(E)-oxim,
4-[17ß-Hydroxy-17α,21-(tert.-butyl)19-nor-3-oxopregna-4,9-dien-20-yn-11β-yl]benzaldehyd-1(E)-oxim,
4-[17β-Hydroxy-17α,21-(4'-tert.-butylphenyl)19-nor-3-oxopregna-4,9-dien-20-yn-11β-yl]benzaldehyd-1 (E)-oxim
4-[17ß-Hydroxy-17α,21-(4'-methylsulfonylphenyl)19nov-3-oxopregna-4,9-dien-20-yn-11β-yl]benzaldehyd-1 (E)-oxim,
4-[17ß-Hydroxy-17a,21-(4'-methylsulfonyl)19-nor-3-oxopregna-4,9-dien-20-yn-11β-yl]benzaldehyd-1(E)-[N-(ethylamin)carbonyl]oxim,
4-[17ß-Hydroxy-17a,21-(4'-methylsulfonylphenyl)19-nor-3-oxopregna-4,9-dien-20-yn-11β-yl]benzaldehyd-1(E)-[S-(ethylthio)carbonyl]oxim,
4-[17β-Hydroxy-17α,21-(4'-methylsulfonylphenyl)19-nor-3-oxopregna-4,9-dien-20-yn-11β-yl]benzaldehyd-1(E)-[O-(ethyloxy)carbonyl]oxim,
4-(17ß-Hydroxy-17a-propinyl-13β-ethyl-3-oxoestra-4,9-dien-11β-yl)benzaldehyd-1(E)-oxim,
4-(6ß-Chlor-17ß-hydroxy-17a-propinyl-3-oxoestra-4,9-dien-11β-yl)benzaldehyd-1(E)-oxim,
4-(7a-Methyl-17ß-hydroxy-17a-propinyl-3-oxoestra-4,9-dien-11β-yl)benzaldehyd-1(E)-oxim,
4-(17β-Acetoxy-17α-propinyl-3-oxoestra-4,9-dien-11β-yl)benzaldehyd-1(E)-oxim und
4-[17β-Acetoxy-17α,21-(4'-methylsulfonylphenyl)19-nor-3-oxopregna-4,9-dien-20-yn-11β-yl]benzaldehyd-1(E)-O-acetyloxim.
4-(17β-Hydroxy-17α-propinyl-3-oxoestra-4,9-dien-11β-yl)benzaldehyd-1(E)-[N-trifluormethoxy phenylamino)carbonyl]oxim

10. Verfahren zur Herstellung der Verbindungen nach einem der Ansprüche 8 oder 9, wobei 11β-Formylphenylsteroide der Formel 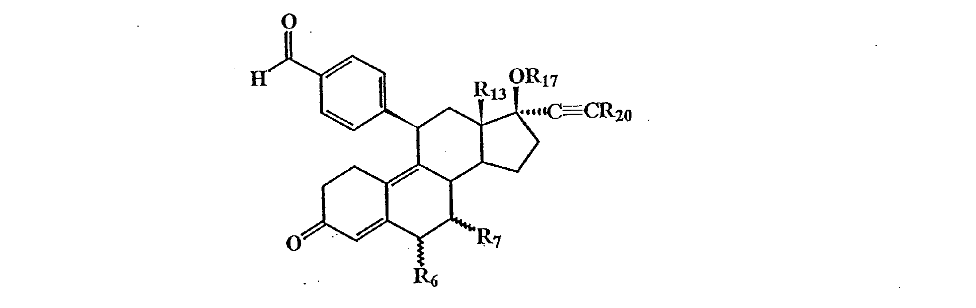 mit Hydroxylamin und/oder Salzen von Hydroxylamin in Lösungsmitteln in Gegenwart einer Base zu den entsprechenden 11β-Benzaldoximen umgesetzt und Hydroxylgruppen gegebenenfalls verestert oder verehtert oder in eine Urethangruppe überführt werden.
